(19) Europäisches Patentamt
European Patent Office
Office européen des brevets

(11) EP 3 013 328 B1

(12) EUROPEAN PATENT SPECIFICATION

(45) Date of publication and mention of the grant of the patent:
**17.10.2018 Bulletin 2018/42**

(21) Application number: **14817963.3**

(22) Date of filing: **27.06.2014**

(51) Int Cl.:
*A61K 9/48* (2006.01) *A61K 31/58* (2006.01)
*A61P 35/00* (2006.01) *A61P 17/14* (2006.01)

(86) International application number:
**PCT/KR2014/005753**

(87) International publication number:
**WO 2014/209062 (31.12.2014 Gazette 2014/53)**

# (54) ORAL SOFT CAPSULE FORMULATION COMPRISING DUTASTERIDE

ORALE WEICHKAPSELFORMULIERUNG MIT DUTASTERID

FORMULE DE CAPSULE MOLLE ORALE COMPRENANT DU DUTASTÉRIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.06.2013 KR 20130075884**
**31.03.2014 KR 20140038291**

(43) Date of publication of application:
**04.05.2016 Bulletin 2016/18**

(73) Proprietor: **Hanmi Pharm. Co., Ltd.**
**Gyeonggi-do 445-910 (KR)**

(72) Inventors:
- **CHO, Jung Hyun**
**Suwon-si**
**Gyeonggi-do 440-841 (KR)**
- **KIM, Jin Cheul**
**Seoul 07216 (KR)**
- **KIM, Yong Il**
**Suwon-si,**
**Gyeonggi-do 16325 (KR)**
- **PARK, Jae Hyun**
**Suwon-si,**
**Gyeonggi-do 16703 (KR)**
- **WOO, Jong Soo**
**Suwon-si**
**Gyeonggi-do 440-710 (KR)**
- **CHOI, Young Keun**
**Suwon-si**
**Gyeonggi-do 440-824 (KR)**

(74) Representative: **Dehns**
**St. Brides House**
**10 Salisbury Square**
**London EC4Y 8JD (GB)**

(56) References cited:
**WO-A1-2010/092596** **WO-A1-2010/124039**
**WO-A2-03/103582** **KR-A- 20090 130 580**
**KR-B1- 101 055 412** **US-A- 5 874 106**

- **Glaxosmithkline: "AVODART SOFT CAPSULES 0.5 MG 1. NAME OF THE MEDICINAL PRODUCT", , 13 March 2013 (2013-03-13), pages 1-16, XP055307788, Retrieved from the Internet: URL:https://health.gsk.sg/content/dam/global/Health/en_SG/pdf/a/avodart_capsule_pi_ipi16si_Poznan_source_approved_18jul13.pdf [retrieved on 2016-10-05]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 013 328 B1

## Description

### FIELD OF THE INVENTION

The present invention relates to an oral soft capsule formulation comprising dutasteride, and more specifically to an oral soft capsule formulation with improved dissolution stability of dutasteride.

### BACKGROUND OF THE INVENTION

Dutasteride (chemical formula: 17β-N-(2,5-bis(trifluoromethyl))phenylcarbamoyl-4-aza-5-α-androst-1-en-3-one), as represented by formula (I) below, is a dual 5-α reductase inhibitor that inhibits conversion of testosterone to dihydrotestosterone (DHT). Dutasteride is known to be useful in the treatment of benign prostatic hyperplasia, prostate cancer, and male pattern alopecia (*see* U.S. Pat. No. 5565467):

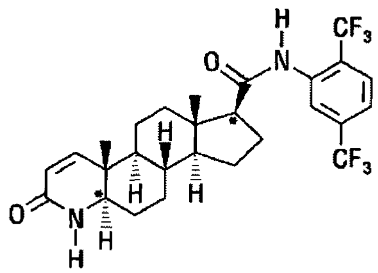

(I).

Self-emulsifying drug delivery systems are being widely used in the process of formulating a drug having low solubility such as dutasteride in order to improve bioavailability of the drug. In this connection, KR Pat. No. 10-1055412 discloses a method for improving the solubility of dutasteride by using a self-emulsifying drug delivery system; and KR. Pat. Laid-open Publication No. 10-2005-0030282 discloses a self-emulsifying drug delivery system as a method of solubilization for improving absorption rate of poorly water-soluble simvastatin by increasing its dissolution rate.

As such, considerable research efforts have been devoted to the methods of solubilization of poorly water-soluble drugs; however, attempts for increasing dissolution rate by improving formulation types for such drugs, which is equally important, have not been made sufficiently.

A formulation type is one of the most important factors in the field of pharmaceutics because application sites and indication may vary with a type of formulation even if same active ingredients are used, and it may also affect pharmacokinetic factors such as absorption rate, etc.

The inventors of the present invention have conducted a research on formulation of dutasteride, and discovered that certain storage conditions caused a reduction in solubility and delay in dissolution of dutasteride even when dutasteride was solubilized by using a self-emulsifying drug delivery system. Gelatin used for making of soft capsules are known to slow down dissolution of a drug due to formation of cross-links among amino acids; however, this phenomenon does not take place in all formulations, but it rather varies depending on the material which is in contact with the gelatins. Specifically, in a soft capsule containing dutasteride, interaction between gelatin and dutasteride promotes cross-linking among amino acids, which causes delay in dissolution. It is thus evident that there is a great need for a novel method which could redress such problems.

The product information "AVODART SOFT CAPSULES 0.5 MG 1. NAME OF THE MEDICINAL PRODUCT" from Glaxosmithkline (2013-03-13, pages 1-16, URL:https://health.gsk.sg/content/dam/global/Health/en_SG/pdf/a/avodart_capsule_pi _ipi16si_Poznan_source_approved_18jul13.pdf) discloses Avodart, which is a dutasteride soft capsule composition comprising gelatin.

WO2010/092596 A1 describes dutasteride compositions encapsulated in a HPMC hard capsule.

KR200901300580A is directed to the composition of a soft capsule shell comprising succinylated gelatine.

### SUMMARY OF THE INVENTION

Therefore, it is an object of the present invention to provide an oral soft capsule formulation, as a self-emulsifying drug delivery system, with improved bioavailability and dissolution stability of dutasteride, and sealability of capsule formulation.

In accordance with one object of the present invention, there is provided an oral soft capsule formulation comprising: (1) a filling material comprising dutasteride; and (2) a soft capsule film containing succinylated gelatin, a

plasticizer, and a cross-linking inhibitor, wherein the succinylated gelatin is comprised in an amount of 60 to 75 wt%, based on the total weight of the soft capsule film.

The oral soft capsule formulation of the present invention filled with dutasteride optimizes the dissolution of dutasteride, and thus can maintain good bioavailability due to consistent dissolution stability, regardless of storage or packaging conditions. Also, the formulation uses suitable materials to improve dissolution stability and sealability of the capsule formulation.

## BRIEF DESCRIPTION OF THE DRAWING

The above and other objects and features of the present invention will become apparent from the following description of the invention, when taken in conjunction with the accompanying drawings.

Fig. 1 shows the comparison of the dissolution profiles of the formulations of Examples 1-H0 and 1-H3, and Comparative Examples 1-H0, 1-H3, 2-H0 and 2-H3.

Fig. 2 shows the comparison of the disintegration times (initial) of the formulations of Example 1 and Comparative Examples 1 and 2 according to the thickness of the film of the formulation.

Fig. 3 shows the comparison of the disintegration times (after storage for one month) of the formulations of Example 1 and Comparative Examples 1 and 2 according to the thickness of the film of the formulation.

Fig. 4A demonstrates a method of cutting a capsule for sealing rate measurement; and Fig. 4B is a picture showing: an upper junction part (DL0), a lower junction part (DL1) and the thickness of the film (DL2).

Fig. 5 shows the sealing rate (in upper junction part) of the formulations of Example 1 and Comparative Examples 1 and 2 according to the thickness of the film of the formulation.

Fig. 6 shows the sealing rate (in lower junction part) of the formulations of Example 1 and Comparative Examples 1 and 2 according to the thickness of the film of the formulation.

## DETAILED DESCRIPTION OF THE INVENTION

The present invention provides an oral soft capsule formulation comprising: (1) a filling material comprising dutasteride; and (2) a soft capsule film containing succinylated gelatin, a plasticizer, and a cross-linking inhibitor, wherein the succinylated gelatin is comprised in an amount of 60 to 75 wt%, based on the total weight of the soft capsule film.

The present invention is explained in detail hereinafter.

### (1) Filling material

The soft capsule formulation according to the present invention comprises dutasteride as an active ingredient. The filling material is preferably an emulsion using a self-emulsifying drug delivery system in order to improve dissolution rate of dutasteride. The filling material may further comprise, besides dutasteride, other ingredients to establish a self-emulsifying drug delivery system. Examples of the other ingredients include a solvent, a surfactant, a stabilizer, and the like. The solvent may be polysorbates including polysorbate 20, 40, 60, and 80; propylene glycol; vegetable oils containing 10 to 20% of ethanol (*e.g.*, peanut oil, safflower oil, olive oil, etc.); glyceryl caprylate/caprate; methyl cellulose; stearoyl monoester of glycerol; monounsaturated fatty acid ester of glycerol; or a mixture thereof. The surfactant may be polysorbates including polysorbate 20, 40, 60, and 80; sorbitan fatty acid esters (*e.g.*, Span® 20, 40, 60, 65, 80, 85); castor oils such as polyoxyl castor oil; substituted castor oils such as Cremophor® or hydrogenated Cremophor®; polyoxyethylene-polyoxypropylene copolymers; mono- or di-glycerides of caprylic/capric acid; polyoxyethylene-polyoxypropylene block copolymer; and the like; preferably poloxamer 407, but not limited thereto. Also, the stabilizer may be selected from the group consisting of water, ethanol, glycine, propylene glycol, polyethylene glycol, diethylene glycol monoethyl ether, dimethyl isosorbide, cetyl alcohol, and a mixture thereof, preferably water, ethanol or glycine. In addition, the emulsion may further comprise other pharmaceutically acceptable additive.

Dutasteride may be comprised in an amount of 0.1 to 1 wt% based on the total weight of the filling material. Also, the solvent may be comprised in an amount of 50 to 93 wt% based on the total weight of the filling material; the surfactant in an amount of 5 to 40 wt% based on the total weight of the filling material; and the stabilizer in an amount of 1 to 10 wt% based on the total weight of the filling material, but not limited thereto.

### (2) Soft capsule film

The film of the soft capsule according to the present invention comprises the following ingredients as its materials.

(a) Succinylated gelatin

Gelatin, which is commonly used as a main ingredient in conventional soft capsules, interacts with dutasteride and promotes cross-linking among amino acids, thereby causing a delay in dissolution of dutasteride. On the contrary, succinylated gelatin does not allow amino acids to form cross-links, and thus can improve dissolution of dutasteride. The succinylated gelatin may be obtained by subjecting gelatin to an alkaline treatment, followed by a reaction with anhydrous succinic acid. As a result, an amino group ($-NH_3^+$) of gelatin undergoes a succinylation, and is substituted with a carboxyl group (-COOH). The succinylated gelatin is used in an amount of 60 to 75 wt%, based on the total weight of the soft capsule film.
A soft capsule formulation comprising the succinylated gelatin of the present invention shows excellent dissolution stability of dutasteride as compared with those of gelatins from pig- or bovine-origin, and it also has advantages in quality maintenance during manufacturing process owing to small changes in sealability and disintegration time of soft capsule formulation in accordance with the thickness of the film.

(b) Plasticizer

In the soft capsule of the present invention, a plasticizer is used to allow the succinylated gelatin to have elasticity so as to prevent hardening of the succinylated gelatin and to remain soft and pliable. Examples of the plasticizer include glycerine, sorbitol, mannitol, propylene glycol, polyethylene glycol, sugar alcohol, monosaccharide, disaccharide, oligosaccharide, etc. and two or more of the plasticizer may be used in combination. The plasticizer may be used in an amount of about 5 to 45 wt%, preferably about 20 to 40 wt%, more preferably about 20 to 37 wt%, based on the total weight of the soft capsule film.

(c) Cross-linking inhibitor

Generally, a cross-linking inhibitor is used to prevent gelatins from forming cross-links. In the present invention, the cross-linking inhibitor prevents the formation of cross-links by interacting with the part of the succinylated gelatins unsubstituted with carboxyl group. Examples of the cross-linking inhibitor may include glycine, citric acid, etc., and two or more of the cross-linking inhibitor may be used in combination. The cross-linking inhibitor may be used in an amount of about 0.5 to 5 wt% based on the total weight of the soft capsule film. When the cross-linking inhibitor is used within said range, desirable sealability and disintegration time of the formulation may be obtained.
The film of the soft capsule of the present invention employing the aforementioned three components improves sealability of the formulation and disintegration time of the drug.
In the preparation of the soft capsule formulation, the thickness of the film is one of the most important parameters which affect product qualities such as disintegration time of the drug and the sealability of the formulation. In consideration of dissolution rate of the drug, disintegration time and the sealability of the formulation, the thickness of the soft capsule formulation comprising dutasteride is preferably about 0.6 mm to 0.8 mm, or about 0.65 mm to 0.75 mm.
However, it may be nearly impossible to completely eliminate the deviation in the thickness of the film no matter how precise the formulation process is, and thus there is a problem of deviation in disintegration time of drug and sealability of formulation.
On the contrary, the soft capsule of the present invention maintains consistency in disintegration time despite having varied film thicknesses, *i.e.*, having deviation in the thickness of the film during the manufacturing process. In one embodiment, the soft capsule of the present invention releases at least 85% of dutasteride as an active ingredient within 15 minutes, and this property is maintained regardless of the type of packaging or the duration of storage. In another embodiment, the oral soft capsule formulation of the present invention releases at least 90% of dutasteride within 30 minutes, and this property is maintained regardless of the packaging type or the duration of storage.
Also, the soft capsule of the present invention shows good sealability of the formulation despite having varied film thicknesses, *i.e.*, having deviation in the thickness of the film during the manufacturing process. Thus, the soft capsule formulation of the present invention can be stored and distributed without getting damaged.
Moreover, the oral soft capsule of the present invention exhibits good dissolution stability against time, and thus can be effectively used in the treatment of benign prostatic hyperplasia, prostate cancer, and male pattern alopecia.
Hereinafter, the present invention is described more specifically by the following examples, but these are provided only for illustration purposes and the present invention is not limited thereto.

**Example 1 and Comparative Examples 1 and 2: Preparation of soft capsule formulations having various types of gelatin**

<Preparation of filling material>

In accordance with the amounts listed in Table 1 below, dutasteride (Dr. Reddy's Laboratories Ltd.) was dissolved in Capmul® MCM oil (Abitech), and the rest of the ingredients were sequentially dissolved so as to obtain a self-emulsifying system.

<Preparation of soft capsule film>

In accordance with the amounts listed in Table 1 below, glycerine, glycine, and distilled water were placed in a gelatin tank, and then uniformly suspended by using a homo mixer. One of succinylated gelatin (Geltech, 200 Bloom), bovine gelatin (Geltech, 165 Bloom), and pig gelatin (Rousselot, 175 Bloom) and distilled water were added to be wetted.

The filling material thus prepared was filled into a soft capsule in accordance with the conventional method described in the General Preparation Chapter of the Korean Pharmacopoeia by using a soft gelatin encapsulation machine (BCM-GB, Bochang Co., Ltd.) to prepare soft capsule formulations of Example 1 and Comparative Examples 1 and 2. During this process, gelatin sheets in the solid state were adjusted before the soft gelatin encapsulation process to maintain the film thickness at 0.8 mm.

[Table 1]

| Component | Ingredient | Amount (mg/formulation) | | |
|---|---|---|---|---|
| | | Example 1 | Comp. Ex. 1 | Comp. Ex. 2 |
| Filling material | Dutasteride | 0.5 | 0.5 | 0.5 |
| | Capmul® MCM | 100 | 100 | 100 |
| | Poloxamer 407 | 10 | 10 | 10 |
| | Distilled water | 3 | 3 | 3 |
| Soft capsule film | Succinylated gelatin | 43.0 | - | - |
| | Bovine gelatin | - | 43.0 | - |
| | Pig gelatin | - | - | 43.0 |
| | Glycerine | 19.0 | 19.0 | 19.0 |
| | Glycine | 0.5 | 0.5 | 0.5 |
| | Distilled water | q.s. | q.s. | q.s. |
| Total | | 176.0 | 176.0 | 176.0 |

**Experimental Example 1: Evaluation of dissolution stability according to gelatin types**

Each of soft capsules obtained in Example 1 and Comparative Examples 1 and 2 were stored under 40°C and 70% RH according to Tables 2, 3, and 4 below.

[Table 2]

| | Ex. 1-H0 | Ex. 1-H1 | Ex. 1-H3 | Ex. 1-P0 | Ex. 1-P1 | Ex. 1-P3 |
|---|---|---|---|---|---|---|
| Sample used | Ex. 1 | Ex. 1 | Ex. 1 | Ex. 1 | Ex. 1 | Ex. 1 |
| Packaging condition | HDPE bottle | HDPE bottle | HDPE bottle | PTP laminated PVDC-Alu | PTP laminated PVDC-Alu | PTP laminated PVDC-Alu |
| Storage duration (month) | 0 | 1 | 3 | 0 | 1 | 3 |

[Table 3]

| | Comp. Ex. 1-H0 | Comp. Ex. 1-H1 | Comp. Ex. 1-H3 | Comp. Ex. 1-P0 | Comp. Ex. 1-P1 | Comp. Ex. 1-P3 |
|---|---|---|---|---|---|---|
| Sample used | Comp. Ex. 1 | Comp. Ex. 1 | Comp. Ex. 1 | Comp. Ex. 1 | Comp. Ex. 1 | Comp. Ex. 1 |
| Packaging condition | HDPE bottle | HDPE bottle | HDPE bottle | PTP laminated PVDC-Alu | PTP laminated PVDC-Alu | PTP laminated PVDC-Alu |
| Storage duration (month) | 0 | 1 | 3 | 0 | 1 | 3 |

[Table 4]

| | Comp. Ex. 2-H0 | Comp. Ex. 2-H1 | Comp. Ex. 2-H3 | Comp. Ex. 2-P0 | Comp. Ex. 2-P1 | Comp. Ex. 2-P3 |
|---|---|---|---|---|---|---|
| Sample used | Comp. Ex. 2 | Comp. Ex. 2 | Comp. Ex. 2 | Comp. Ex. 2 | Comp. Ex. 2 | Comp. Ex. 2 |
| Packaging condition | HDPE bottle | HDPE bottle | HDPE bottle | PTP laminated PVDC-Alu | PTP laminated PVDC-Alu | PTP laminated PVDC-Alu |
| Storage duration (month) | 0 | 1 | 3 | 0 | 1 | 3 |

As shown in Tables above, PVDC-Alu refers to a blister pack which consists of a lidding seal of polyvinylidene chloride (PVDC) and a backing of aluminum foil. Also, Example 1-H0 refers to the sample in which the soft capsule of Example 1 was packaged in a high-density polyethylene (HDPE) bottle and stored for 0 month; Example 1-H1 refers to the sample in which the soft capsule of Example 1 was packaged in an HDPE bottle and stored for 1 month; Example 1-H3 refers to the sample in which the soft capsule of Example 1 was packaged in an HDPE bottle and stored for 3 months; Example 1-P0 refers to the sample in which the soft capsule of Example 1 was packaged in a press through package (PTP) and stored for 0 month; Example 1-P1 refers to the sample in which the soft capsule of Example 1 was packaged in a PTP and stored for 1 month; and Example 1-P3 refers to the sample in which the soft capsule of Example 1 was packaged in a PTP and stored for 3 months. The samples of Comparative Examples 1 and 2 were named in the same manner as Example 1.

The soft capsule samples were subjected to the dissolution test using 2% SLS dissolved in 0.1 N HCl solution (900 mL) as an eluent at a rate of 50 rpm, as described in Dissolution Apparatus 2 (paddle) of the United States Pharmacopeia. The dissolution rates of the samples were analyzed by collecting the samples at 5, 15, 30, 45 and 60 minutes after the test and measuring them. The results are shown in Tables 5 to 7, and Fig. 1.

[Table 5]

| Sampling time (min) | Ex. 1-H0 | Ex. 1-H1 | Ex. 1-H3 | Ex. 1-P0 | Ex. 1-P1 | Ex. 1-P3 |
|---|---|---|---|---|---|---|
| 5 | 3.0 | 0.0 | 0.0 | 5.0 | 0.0 | 1.7 |
| 15 | 92.2 | 91.8 | 89.5 | 92.8 | 92.4 | 90.8 |
| 30 | 98.0 | 96.5 | 95.4 | 97.2 | 98.1 | 96.4 |
| 45 | 99.1 | 98.1 | 98.2 | 97.8 | 98.2 | 96.5 |
| 60 | 98.8 | 98.6 | 98.5 | 98.1 | 98.1 | 97.5 |

[Table 6

| Sampling time (min) | Comp. Ex. 1-H0 | Comp. Ex. 1-H1 | Comp. Ex. 1-H3 | Comp. Ex. 1-P0 | Comp. Ex. 1-P1 | Comp. Ex. 1-P3 |
|---|---|---|---|---|---|---|
| 5 | 0.0 | 0.0 | 0.0 | 0.0 | 0.0 | 0.0 |
| 15 | 82.7 | 43.5 | 20.2 | 88.5 | 61.5 | 0.0 |
| 30 | 94.5 | 70.3 | 39.1 | 89.8 | 83.2 | 77.6 |
| 45 | 96.4 | 89.4 | 71.1 | 94.5 | 96.4 | 96.3 |
| 60 | 98.4 | 97.9 | 98.2 | 97.9 | 98.2 | 98.4 |

[Table 7]

| Sampling time (min) | Comp. Ex. 2-H0 | Comp. Ex. 2-H1 | Comp. Ex. 2-H3 | Comp. Ex. 2-P0 | Comp. Ex. 2-P1 | Comp. Ex. 2-P3 |
|---|---|---|---|---|---|---|
| 5 | 0.0 | 0.0 | 0.0 | 0.0 | 0.0 | 0.0 |
| 15 | 86.5 | 38.1 | 0.0 | 84.5 | 0.8 | 1.8 |
| 30 | 95.7 | 60.2 | 28.4 | 93.4 | 72.8 | 42.0 |
| 45 | 96.2 | 82.4 | 51.6 | 97.2 | 90.5 | 65.4 |
| 60 | 99.4 | 97.9 | 98.0 | 97.9 | 98.1 | 97.2 |

As shown in Tables 5 to 7 and Fig. 1, Example 1 having succinylated gelatin as the soft capsule material exhibited consistent dissolution rates regardless of the type of packaging or duration of storage. On the other hand, Comparative Examples 1 and 2 having pig gelatin or bovine gelatin as the soft capsule material showed, although there were some differences depending on the type of packaging, a reduction in dissolution rate as time passes.

**Examples 1-1 to 1-5, Comparative Examples 1-1 to 1-5, and Comparative Examples 2-1 to 2-5: Preparation of soft capsule formulations having various types of gelatin and various thickness of soft capsule film**

In order to evaluate the effect of the type of gelatin on the soft capsules having various film thicknesses, the film thickness of the soft capsule formulations obtained in Example 1 and Comparative Examples 1 and 2 was varied according to Table 8 below. The film thickness was adjusted to 0.6, 0.65, 0.7, 0.75, and 0.8 mm by changing the thickness of gelatin sheets before the soft gelatin encapsulation process. Soft capsule formulations having various thicknesses were prepared and named Examples 1-1 to 1-5, Comparative Examples 1-1 to 1-5 and 2-1 to 2-5. Example 1-1 refers to a formulation of Example 1 having the film thickness of 0.6 mm; Example 1-2 refers to a formulation of Example 1 having the film thickness of 0.65 mm; Example 1-3 refers to a formulation of Example 1 having the film thickness of 0.7 mm; Example 1-4 refers to a formulation of Example 1 having the film thickness of 0.75 mm; and Example 1-5 refers to a formulation of Example 1 having the film thickness of 0.8 mm. Likewise, each of Comparative Examples 1-1 to 1-5 and 2-1 to 2-5 has the thicknesses as indicated in Table 8 below.

[Table 8]

| | Ex. 1-1 | Ex. 1-2 | Ex. 1-3 | Ex. 1-4 | Ex. 1-5 |
|---|---|---|---|---|---|
| Formulation | Example 1 | | | | |
| Film thickness(mm) | 0.6 | 0.65 | 0.7 | 0.75 | 0.8 |
| | | | | | |
| | Comp. Ex. 1-1 | Comp. Ex. 1-2 | Comp. Ex. 1-3 | Comp. Ex. 1-4 | Comp. Ex. 1-5 |
| Formulation | Comparative Example 1 | | | | |
| Film thickness(mm) | 0.6 | 0.65 | 0.7 | 0.75 | 0.8 |
| | | | | | |

(continued)

| | Comp. Ex. 2-1 | Comp. Ex. 2-2 | Comp. Ex. 2-3 | Comp. Ex. 2-4 | Comp. Ex. 2-5 |
|---|---|---|---|---|---|
| Formulation | Comparative Example 2 | | | | |
| Film thickness(mm) | 0.6 | 0.65 | 0.7 | 0.75 | 0.8 |

**Experimental Example 2: Evaluation of disintegration time according to gelatin types and thickness of film**

Soft capsule formulations of Examples 1-1 to 1-5, Comparative Examples 1-1 to 1-5 and 2-1 to 2-5, which have various gelatin types and thicknesses of film, were each packaged in an HDPE bottle and stored under 40°C and 70%, and then disintegration time of each formulation was evaluated according to the disintegration test method described in the Korean Pharmacopoeia. Specifically, samples were decided as disintegrated and disintegration time was determined when there was no sample residue remaining in a glass tube or there remained some sample in a glass tube yet it was a soft material without having a distinct circle shape, or it was a fragment of insoluble coating or coating film. The results are shown in Tables 9 to 11 and Figs. 2 and 3.

[Table 9]

| | | Ex. 1-1 | Ex. 1-2 | Ex. 1-3 | Ex. 1-4 | Ex. 1-5 |
|---|---|---|---|---|---|---|
| Film thickness (mm) | | 0.6 | 0.65 | 0.7 | 0.75 | 0.8 |
| Disintegration time (initial) | 1st trial | 2.8 | 3.5 | 3.5 | 3.8 | 4.6 |
| | 2nd trial | 2.6 | 4.2 | 3.2 | 4.0 | 4.8 |
| | 3rd trial | 3.2 | 3.0 | 4.0 | 4.2 | 4.4 |
| | Avg (min) | 2.87 | 3.57 | 3.57 | 4.0 | 4.6 |
| Disintegration time (after storage for 1 month) | 1st trial | 3.0 | 3.8 | 4.8 | 5.0 | 10.0 |
| | 2nd trial | 3.6 | 6.5 | 6.0 | 6.2 | 12.5 |
| | 3rd trial | 4.8 | 3.5 | 4.0 | 6.8 | 14.8 |
| | Avg (min) | 3.8 | 4.6 | 4.93 | 6.0 | 12.43 |

[Table 10]

| | | Comp. Ex. 1-1 | Comp. Ex. 1-2 | Comp. Ex. 1-3 | Comp. Ex. 1-4 | Comp. Ex. 1-5 |
|---|---|---|---|---|---|---|
| Film thickness (mm) | | 0.6 | 0.65 | 0.7 | 0.75 | 0.8 |
| Disintegration time (initial) | 1st trial | 1.0 | 3.0 | 3.6 | 4.8 | 12.6 |
| | 2nd trial | 3.2 | 2.8 | 3.8 | 5.2 | 10.8 |
| | 3rd trial | 2.2 | 2.4 | 3.2 | 8.0 | 9.6 |
| | Avg (min) | 2.13 | 2.73 | 3.53 | 6.00 | 11.00 |

(continued)

| | | Comp. Ex. 1-1 | Comp. Ex. 1-2 | Comp. Ex. 1-3 | Comp. Ex. 1-4 | Comp. Ex. 1-5 |
|---|---|---|---|---|---|---|
| Disintegration time (after storage for 1 month) | 1st trial | 9.0 | 15.0 | 14.3 | 16.2 | 20.5 |
| | 2nd trial | 8.0 | 10.3 | 16.5 | 20.6 | 16.8 |
| | 3rd trial | 8.8 | 11.6 | 17.0 | 17.4 | 14.0 |
| | Avg (min) | 8.60 | 12.30 | 15.93 | 18.07 | 17.10 |

[Table 11]

| | | Comp. Ex. 2-1 | Comp. Ex. 2-2 | Comp. Ex. 2-3 | Comp. Ex. 2-4 | Comp. Ex. 2-5 |
|---|---|---|---|---|---|---|
| Film thickness (mm) | | 0.6 | 0.65 | 0.7 | 0.75 | 0.8 |
| Disintegration time (initial) | 1st trial | 1.5 | 3.6 | 4.0 | 6.0 | 13.2 |
| | 2nd trial | 3.5 | 3.2 | 4.0 | 8.6 | 8.0 |
| | 3rd trial | 2.0 | 4.0 | 4.2 | 9.0 | 9.2 |
| | Avg (min) | 2.33 | 3.60 | 4.07 | 7.87 | 10.13 |
| Disintegration time (after storage for 1 month) | 1st trial | 10.8 | 16.4 | 16.5 | 16.0 | 18.5 |
| | 2nd trial | 8.5 | 13.5 | 14.8 | 18.0 | 16.4 |
| | 3rd trial | 12.0 | 10.8 | 15.0 | 18.5 | 20.0 |
| | Avg (min) | 10.43 | 13.57 | 15.43 | 17.50 | 18.30 |

As shown in Tables 9 to 11 above, Examples 1-1 to 1-5 containing succinylated gelatin exhibited small changes in disintegration time among formulations with various film thicknesses for both the initial and after storage for one month. Contrarily, Comparative Examples 1-1 to 1-5 and 2-1 to 2-5 containing either bovine or pig gelatin resulted in a large difference in disintegration time depending on the film thickness of the formulation.

In the preparation of the soft capsule formulation, the thickness of the film is one of the most important parameters which affect product qualities, and thus it is advantageous to have a broader tolerable range of such parameter during the preparation process. It can be shown that consistency in disintegration time is maintained in a larger range of film thickness when succinylated gelatin is used as a film material as compared with other types of gelatin. That is to say, the deviation of the thickness, which might occur during the preparation process, does not greatly affect the product qualities when succinylated gelatin is used as a film material.

**Experimental Example 3: Evaluation of sealability of soft capsule according to gelatin types and thickness of film**

In a soft capsule formulation, sealability is an important factor which is directly related to product quality. Generally, a film of a soft capsule is formed by sealing two sheets; so if sealing is not formed appropriately, the film is easily damaged during the storage and distribution of the formulation. Also, the sealability may change significantly when

there is a deviation of film thickness in the preparation of formulation. Thus, it is an important task in the field of pharmaceutics to maintain a desirable sealability of a soft capsule formulation regardless of its film thickness.

Accordingly, sealability of soft capsules having various gelatin types and film thicknesses was evaluated so as to examine the effect of gelatin types on sealabilities of soft capsules having various film thicknesses.

Specifically, a midsection of the soft capsule formulations of Examples 1-1 to 1-5, Comparative Examples 1-1 to 1-5 and 2-1 to 2-5 which have various gelatin types and thicknesses of film was cut (*see* Fig. 4A), and the filling materials were removed therefrom. Then, the thickness of the film (DL2) and the thickness of the thinnest parts of the film, *i.e.*, in an upper junction part (DL0) and a lower junction part (DL1), were measured (*see* Fig. 4B), and sealability of the upper and lower junction parts were measured using the equations below:

- $$\text{Sealing rate (upper part) (\%)} = (DL0 / DL2) \times 100$$

- $$\text{Sealing rate (lower part) (\%)} = (DL1 / DL2) \times 100$$

The sealing rate measurements are shown in Tables 12 to 14 and Figs. 5 and 6.

[Table 12]

| | Ex. 1-1 | Ex. 1-2 | Ex. 1-3 | Ex. 1-4 | Ex. 1-5 |
|---|---|---|---|---|---|
| Film thickness (mm) | 0.6 | 0.65 | 0.7 | 0.75 | 0.8 |
| Sealing rate of upper part (%) | 55.8 | 72.3 | 80.4 | 88.6 | 90.2 |
| Sealing rate of lower part (%) | 74.6 | 81.7 | 94.5 | 93.5 | 95.6 |

[Table 13]

| | Comp. Ex. 1-1 | Comp. Ex. 1-2 | Comp. Ex. 1-3 | Comp. Ex. 1-4 | Comp. Ex. 1-5 |
|---|---|---|---|---|---|
| Film thickness (mm) | 0.6 | 0.65 | 0.7 | 0.75 | 0.8 |
| Sealing rate of upper part (%) | 31.6 | 48.6 | 78.3 | 79.4 | 81.7 |
| Sealing rate of lower part (%) | 50.8 | 59.6 | 80.1 | 86.2 | 92.5 |

[Table 14]

| | Comp. Ex. 2-1 | Comp. Ex. 2-2 | Comp. Ex. 2-3 | Comp. Ex. 2-4 | Comp. Ex. 2-5 |
|---|---|---|---|---|---|
| Film thickness (mm) | 0.6 | 0.65 | 0.7 | 0.75 | 0.8 |
| Sealing rate of upper part (%) | 28.5 | 53 | 72.4 | 83.5 | 86.3 |
| Sealing rate of lower part (%) | 53.6 | 70 | 77.8 | 89.4 | 94.5 |

As can be seen in Tables above and Figs., Examples 1-1 to 1-5 containing succinylated gelatin exhibited small changes in sealing rate among formulations with various film thicknesses. Contrarily, Comparative Examples 1-1 to 1-5 and 2-1 to 2-5 containing either bovine or pig gelatin resulted in a large difference in sealing rate depending on the thicknesses of the film.

In the preparation of a soft capsule formulation, the thickness of the film is one of the most important parameters which affect product qualities, and thus it is advantageous to have a broader range of such parameter during the preparation process. It can be shown that consistency in sealing rate is maintained in a larger range of film thickness when succinylated gelatin is used as a film material as compared with other types of gelatin. That is to say, the deviation of the thickness, which might occur during the preparation process, does not greatly affect the product qualities when succinylated gelatin is used as a film material.

**Examples 2 to 5: Preparation of soft capsule formulations having various amounts of cross-linking inhibitor**

In order to evaluate the effect of the cross-linking inhibitor on the soft capsule formulations, soft capsule formulations with various amounts of cross-linking inhibitor, (*i.e.*, glycine) were prepared according to Table 15 below.

[Table 15]

| Component | Ingredient | Amount (mg/formulation) | | | | |
|---|---|---|---|---|---|---|
| | | Ex. 2 | Ex. 3 | Ex. 1 | Ex. 4 | Ex. 5 |
| Filling material | Dutasteride | 0.5 | 0.5 | 0.5 | 0.5 | 0.5 |
| | Capmul® MCM | 100 | 100 | 100 | 100 | 100 |
| | Poloxamer 407 | 10 | 10 | 10 | 10 | 10 |
| | Distilled water | 3 | 3 | 3 | 3 | 3 |
| Soft capsule film | Succinylated gelatin | 43.0 | 43.0 | 43.0 | 43.0 | 43.0 |
| | Glycerine | 19.0 | 19.0 | 19.0 | 19.0 | 19.0 |
| | Glycine | 0.1 | 0.3 | 0.5 | 2.0 | 5.0 |
| | Distilled water | q.s. | q.s. | q.s. | q.s. | q.s. |
| Total | | 175.6 | 175.8 | 176.0 | 177.5 | 180.5 |
| Amount of glycine in the soft capsule film (%) | | 0.16 | 0.48 | 0.80 | 3.13 | 7.46 |

**Experimental Example 4: Evaluation of disintegration time according to amounts of cross-linking inhibitor**

Soft capsule formulations of Examples 1 to 5 were separately packaged in an HDPE bottle and stored for one month under 40°C and 70% RH, and then disintegration time of each formulation was evaluated according to the method described in Experimental Example 2. The results are shown in Table 16 below.

[Table 16]

| Disintegration time | | Ex. 2 | Ex. 3 | Ex. 1 | Ex. 4 | Ex. 5 |
|---|---|---|---|---|---|---|
| Initial | 1st trial | 5.2 | 4.0 | 4.6 | 4.0 | 2.5 |
| | 2nd trial | 3.5 | 5.0 | 4.8 | 3.8 | 3.8 |
| | 3rd trial | 4.2 | 3.8 | 4.4 | 3.8 | 3.6 |
| | Avg (min) | 4.3 | 4.3 | 4.6 | 3.9 | 3.3 |
| After storage for 1 month | 1st trial | 16.6 | 9.5 | 5.4 | 4.0 | 3.6 |
| | 2nd trial | 10.2 | 13.5 | 3.8 | 3.0 | 1.8 |
| | 3rd trial | 9.0 | 6.8 | 3.8 | 6.2 | 2.0 |
| | Avg (min) | 11.9 | 9.9 | 4.3 | 4.4 | 2.5 |

As shown in Table 16 above, Examples 2 and 3 which contained glycine as a cross-linking inhibitor in amounts of, respectively, 0.1 mg (0.16 wt%) and 0.3 mg (0.48 wt%) showed a considerable increase in disintegration time due to insufficient inhibitory activity against the formation of cross-links when stored under accelerated conditions for 1 month. Thus, it can be concluded that the amount of glycine as the cross-linking inhibitor is preferably at least 0.50 wt%, based on the total weight of the soft capsule film, for maintaining consistency in disintegration time against storage time.

**Experimental Example 5: Evaluation of sealability of soft capsules according to amounts of cross-linking inhibitor**

The following test was conducted in order to evaluate the effects of the amounts of cross-linking inhibitor on sealability of soft capsules. For precise measurement, soft capsules of Examples 1 to 5 with the film thickness of 0.8

mm were prepared. Then, sealing rates of the upper and lower junctions were measured according to the method as described in Experimental Example 3. The sealing rate measurements are shown in Table 17 below.

[Table 17]

| Sealing rate (%) | Ex. 2 | Ex. 3 | Ex. 1 | Ex. 4 | Ex. 5 |
|---|---|---|---|---|---|
| Upper part | 92.4 | 89.5 | 90.2 | 73.6 | 36.8 |
| Lower part | 92.8 | 94.1 | 95.6 | 86.1 | 65.4 |

As shown in Table 17 above, the sealing rates decreased as the amount of glycine as the cross-linking inhibitor increased because glycine negatively affected the properties of the capsule film. Particularly, the sealing rate of Example 5 containing glycine in an amount of 7.46 wt% significantly decreased as compared with those of Examples 1 to 4 containing glycine in amounts of 0.16 to 3.13 wt%. Thus, in consideration of sealing rate, the amount of glycine to be used is preferably 5 wt% at most.

**Examples 6 to 9: Preparation of soft capsule formulations having various amounts of succinylated gelatin**

In order to evaluate the effect of the succinylated gelatin on the soft capsule formulations, soft capsule formulations were prepared based on Example 1, varying the amount of succinylated gelatin according to Table 18 below.

[Table 18]

| Component | Ingredient | Amount (mg/formulation) | | | | |
|---|---|---|---|---|---|---|
| | | Ex. 6 | Ex. 7 | Ex. 1 | Ex. 8 | Ex. 9 |
| Filling material | Dutasteride | 0.5 | 0.5 | 0.5 | 0.5 | 0.5 |
| | Capmul® MCM | 100 | 100 | 100 | 100 | 100 |
| | Poloxamer 407 | 10 | 10 | 10 | 10 | 10 |
| | Distilled water | 3 | 3 | 3 | 3 | 3 |
| Soft capsule film | Succinylated gelatin | 23.0 | 33.0 | 43.0 | 53.0 | 63.0 |
| | Glycerine | 19.0 | 19.0 | 19.0 | 19.0 | 19.0 |
| | Glycine | 0.5 | 0.5 | 0.5 | 0.5 | 0.5 |
| | Distilled water | q.s. | q.s. | q.s. | q.s. | q.s. |
| Total amount | | 156.0 | 166.0 | 176.0 | 186.0 | 196.0 |
| Amount of gelatin in the soft capsule film (%) | | 54.12 | 62.86 | 68.80 | 73.10 | 76.36 |
| Amount of glycine in the soft capsule film (%) | | 1.18 | 0.95 | 0.80 | 0.69 | 0.61 |

**Experimental Example 6: Evaluation of disintegration time according to amounts of succinylated gelatin**

Soft capsule formulations of Examples 1 and 6 to 9 were separately packaged in an HDPE bottle and stored for one month under 40°C and 70% RH, and then disintegration time of each formulation was evaluated according to the method described in Experimental Example 2. The results are shown in Table 19 below.

[Table 19]

| Disintegration time | | Ex. 6 | Ex. 7 | Ex. 1 | Ex. 8 | Ex. 9 |
|---|---|---|---|---|---|---|
| Initial | 1st trial | 1.8 | 4.0 | 4.6 | 4.5 | 10.6 |
| | 2nd trial | 3.2 | 5.6 | 4.8 | 8.5 | 5.6 |
| | 3rd trial | 3.0 | 1.6 | 4.4 | 5.2 | 9.5 |
| | Avg (min) | 2.7 | 3.7 | 4.6 | 6.1 | 8.6 |

(continued)

| Disintegration time | | Ex. 6 | Ex. 7 | Ex. 1 | Ex. 8 | Ex. 9 |
|---|---|---|---|---|---|---|
| After storage for 1 month | 1st trial | 3.0 | 6.0 | 5.4 | 6.0 | 16.0 |
| | 2nd trial | 3.2 | 6.2 | 3.8 | 6.5 | 14.5 |
| | 3rd trial | 1.5 | 4.0 | 3.8 | 8.2 | 14.2 |
| | Avg (min) | 2.6 | 5.4 | 4.3 | 6.9 | 14.9 |

As shown in Table 19 above, the formulation of Example 9 which contained succinylated gelatin in an amount of 76.36 wt% showed a considerable increase in disintegration time when stored under the accelerated conditions for 1 month despite the fact that the amount of glycine as the cross-linking inhibitor was within the preferable amount range, *i.e.*, 0.50 to 5.0 wt%. Thus, it can be concluded that the amount of the succinylated gelatin to be used is preferably 75 wt% at most, based on the total weight of the soft capsule film, for maintaining consistency in disintegration time against storage time.

**Experimental Example 7: Evaluation of sealability of soft capsules according to amounts of succinylated gelatin**

The following test was conducted in order to evaluate the effects of the amounts of succinylated gelatin on sealability of soft capsules. For precise measurement, soft capsules of Examples 1 and 6 to 9 with the film thickness of 0.8 mm were prepared. Then, sealing rates of the upper and lower junction parts were measured according to the method as described in Experimental Example 3. The sealing rate measurements are shown in Table 20 below.

[Table 20]

| Sealing rate (%) | Ex. 6 | Ex. 7 | Ex. 1 | Ex. 8 | Ex. 9 |
|---|---|---|---|---|---|
| Upper part | 26.5 | 79.2 | 90.2 | 89.5 | 91.8 |
| Lower part | 56.8 | 90.6 | 95.6 | 96.8 | 95.5 |

As shown in Table 20 above, the sealing rates of the formulations of Examples 1 and 7 to 9 which contained succinylated gelatin in amounts of at least 62.86 wt% were good; whereas the sealing rate of the formulation of Example 6 which contained succinylated gelatin in an amount of 54.12 wt% was significantly low. Accordingly, in consideration of sealing rate, the amount of the succinylated gelatin to be used is preferably at least 60 wt%.

## Claims

**1.** An oral soft capsule formulation comprising:

(1) a filling material comprising dutasteride; and
(2) a soft capsule film containing succinylated gelatin, a plasticizer, and a cross-linking inhibitor, wherein

the succinylated gelatin is comprised in an amount of 60 to 75 wt%, based on the total weight of the soft capsule film.

**2.** The oral soft capsule formulation of claim 1, wherein the filling material is an emulsion.

**3.** The oral soft capsule formulation of claim 1, wherein the filling material contains a solvent selected from the group consisting of polysorbates, propylene glycol, vegetable oils containing 10 to 20% of ethanol, glyceryl caprylate/caprate, methyl cellulose, stearoyl monoester of glycerol, monounsaturated fatty acid ester of glycerol, and a mixture thereof.

**4.** The oral soft capsule formulation of claim 1, wherein the filling material contains a surfactant selected from the group consisting of polysorbates, sorbitan fatty acid esters, castor oils, substituted castor oils, polyoxyethylene-polyoxypropylene copolymers, mono- or di-glycerides of caprylic/capric acid, polyoxyethylene-polyoxypropylene block copolymer, and a mixture thereof.

5. The oral soft capsule formulation of claim 1, wherein the filling material contains a stabilizer selected from the group consisting of water, ethanol, glycine, propylene glycol, polyethylene glycol, diethylene glycol monoethyl ether, dimethyl isosorbide, cetyl alcohol, and a mixture thereof.

6. The oral soft capsule formulation of claim 1, wherein the plasticizer is selected from the group consisting of glycerine, sorbitol, mannitol, propylene glycol, polyethylene glycol, sugar alcohol, monosaccharide, disaccharide, oligosaccharide, and a mixture thereof.

7. The oral soft capsule formulation of claim 1, wherein the plasticizer is comprised in an amount of 5 to 45 wt%, based on the total weight of the soft capsule film.

8. The oral soft capsule formulation of claim 1, wherein the cross-linking inhibitor is selected from the group consisting of glycine, citric acid, and a mixture thereof.

9. The oral soft capsule formulation of claim 1, wherein the cross-linking inhibitor is comprised in an amount of 0.5 to 5 wt%, based on the total weight of the soft capsule film.

10. The oral soft capsule formulation of claim 1, wherein the soft capsule releases at least 85% of dutasteride within 15 minutes.

11. The oral soft capsule formulation of claim 10, wherein the soft capsule releases at least 90% of dutasteride within 30 minutes.

## Patentansprüche

1. Orale Weichkapsel-Formulierung umfassend:

   (1) ein Füllmaterial umfassend Dutasterid; und
   (2) einen Weichkapsel-Überzug enthaltend succinylierte Gelatine, einen Weichmacher und einen Vernetzungsinhibitor,

   wobei
   die succinylierte Gelatine basierend auf dem Gesamtgewicht des Weichkapsel-Überzugs in einer Menge von 60 bis 75 Gew.-% umfasst ist.

2. Orale Weichkapsel-Formulierung nach Anspruch 1, wobei das Füllmaterial eine Emulsion ist.

3. Orale Weichkapsel-Formulierung nach Anspruch 1, wobei das Füllmaterial ein Lösungsmittel enthält, ausgewählt aus der Gruppe bestehend aus Polysorbaten, Propylenglykol, Pflanzenölen enthaltend 10 bis 20% an Ethanol, Glycerin-Caprylate/Caprate, Methylcellulose, Steaorylmonoester des Glycerins, einfach ungesättigte Fettsäuren des Glycerins und eine Mischung davon.

4. Orale Weichkapsel-Formulierung nach Anspruch 1, wobei das Füllmaterial ein Tensid enthält, ausgewählt aus der Gruppe bestehend aus Polysorbaten, Sorbitan-Fettsäurestern, Castorölen, substituierten Castorölen, Polyoxyethlyen-Polyoxypropylen Copolymeren, Mono- oder Diglyceriden von Caprylsäure/Caprinsäure, Polyoxyethlyen-Polyoxypropylen Blockcopolymeren und eine Mischung davon.

5. Orale Weichkapsel-Formulierung nach Anspruch 1, wobei das Füllmaterial einen Stabilisator enthält, ausgewählt aus der Gruppe bestehend aus Wasser, Ethanol, Glycin, Propylenglykol, Polyethylenglycol, Diethylenglykolmonoethylether, Dimethylisosorbid, Cetylalkohol und eine Mischung davon.

6. Orale Weichkapsel-Formulierung nach Anspruch 1, wobei der Weichmacher ausgewählt ist aus der Gruppe bestehend aus Glycerin, Sorbitol, Mannitol, Propylenglykol, Polyethylenglykol, Zuckeralkohol, Monosaccharid, Disaccharid, Oligosaccharid und eine Mischung davon.

7. Orale Weichkapsel-Formulierung nach Anspruch 1, wobei der Weichmacher basierend auf dem Gesamtgewicht des Weichkapsel-Überzugs in einer Menge von 5 bis 45 Gew.-% umfasst ist.

8. Orale Weichkapsel-Formulierung nach Anspruch 1, wobei der Vernetzungsinhibitor ausgewählt ist aus der Gruppe bestehend aus Glycin, Zitronensäure und eine Mischung davon.

9. Orale Weichkapsel-Formulierung nach Anspruch 1, wobei der Vernetzungsinhibitor basierend auf dem Gesamtgewicht des Weichkapsel-Überzugs in einer Menge von 0,5 bis 5 Gew.-% umfasst ist.

10. Orale Weichkapsel-Formulierung nach Anspruch 1, wobei die Weichkapsel innerhalb von 15 Minuten mindestens 85 % des Dutasterids freisetzt.

11. Orale Weichkapsel-Formulierung nach Anspruch 10, wobei die Weichkapsel innerhalb von 30 Minuten mindestens 90 % des Dutasterids freisetzt.

## Revendications

1. Formulation de capsule molle orale comprenant :

   (1) un matériau de remplissage comprenant du dutastéride ; et
   (2) un film de capsule molle contenant de la gélatine succinylée, un plastifiant, et un inhibiteur de réticulation,

   dans laquelle
   la gélatine succinylée est comprise dans une quantité de 60 à 75 % en poids, en se basant sur le poids total du film de capsule molle.

2. Formulation de capsule molle orale selon la revendication 1, dans laquelle le matériau de remplissage est une émulsion.

3. Formulation de capsule molle orale selon la revendication 1, dans laquelle le matériau de remplissage contient un solvant choisi dans le groupe constitué de polysorbates, de propylène glycol, d'huiles végétales contenant 10 à 20 % d'éthanol, de caprylate/caprate de glycéryle, de méthylcellulose, de monoester stéaroylique de glycérol, d'ester d'acide gras monoinsaturé de glycérol, et d'un mélange de ceux-ci.

4. Formulation de capsule molle orale selon la revendication 1, dans laquelle le matériau de remplissage contient un tensioactif choisi dans le groupe constitué de polysorbates, d'esters d'acides gras de sorbitane, d'huiles de ricin, d'huiles de ricin substituées, de copolymères polyoxyéthylène-polyoxypropylène, de mono- ou de di-glycérides d'acide caprylique/caprique, de copolymère séquencé polyoxyéthylène-polyoxypropylène, et d'un mélange de ceux-ci.

5. Formulation de capsule molle orale selon la revendication 1, dans laquelle le matériau de remplissage contient un stabilisant choisi dans le groupe constitué d'eau, d'éthanol, de glycine, de propylène glycol, de polyéthylène glycol, d'éther monoéthylique de diéthylène glycol, d'isosorbide de diméthyle, d'alcool cétylique, et d'un mélange de ceux-ci.

6. Formulation de capsule molle orale selon la revendication 1, dans laquelle le plastifiant est choisi dans le groupe constitué de glycérine, de sorbitol, de mannitol, de propylène glycol, de polyéthylène glycol, d'alcool de sucre, de monosaccharide, de disaccharide, d'oligosaccharide, et d'un mélange de ceux-ci.

7. Formulation de capsule molle orale selon la revendication 1, dans laquelle le plastifiant est compris en une quantité de 5 à 45 % en poids, en se basant sur le poids total du film de capsule molle.

8. Formulation de capsule molle orale selon la revendication 1, dans laquelle l'inhibiteur de réticulation est choisi dans le groupe constitué de glycine, d'acide citrique, et d'un mélange de ceux-ci.

9. Formulation de capsule molle orale selon la revendication 1, dans laquelle l'inhibiteur de réticulation est compris en une quantité de 0,5 à 5% en poids, en se basant sur le poids total du film de capsule molle.

10. Formulation de capsule molle orale selon la revendication 1, dans laquelle la capsule molle libère au moins 85 % du dutastéride en 15 minutes.

11. Formulation de capsule molle orale selon la revendication 10, dans laquelle la capsule molle libère au moins 90% du dutastéride en 30 minutes.

**Fig. 1**

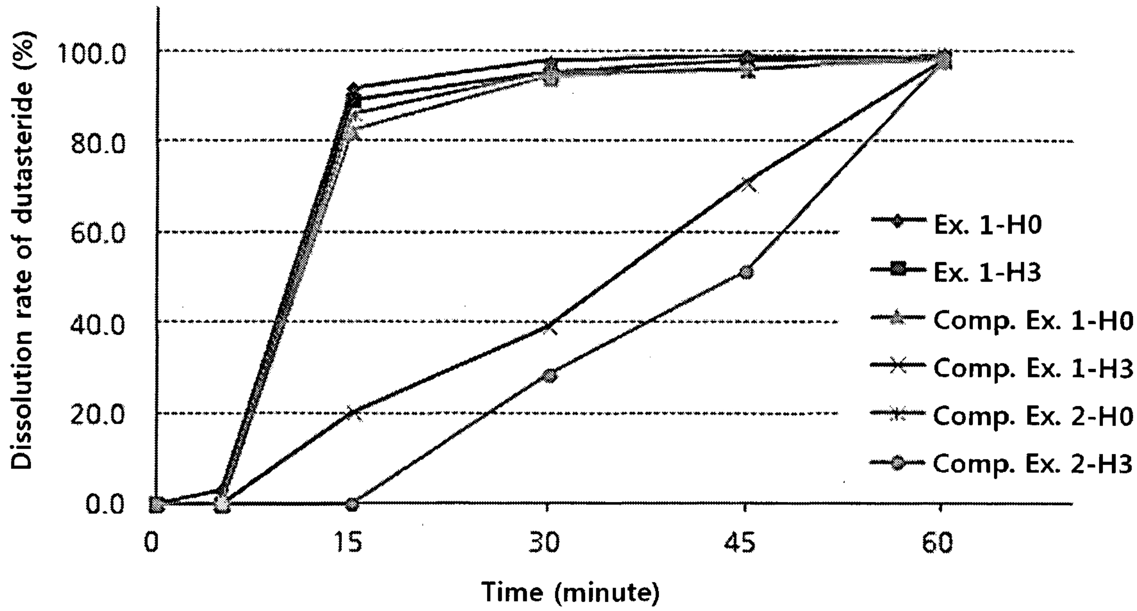
Dissolution rate of dutasteride (%)
100.0
80.0
60.0
40.0
20.0
0.0
0
15
30
45
60
Time (minute)
Ex. 1-H0
Ex. 1-H3
Comp. Ex. 1-H0
Comp. Ex. 1-H3
Comp. Ex. 2-H0
Comp. Ex. 2-H3

**Fig. 2**

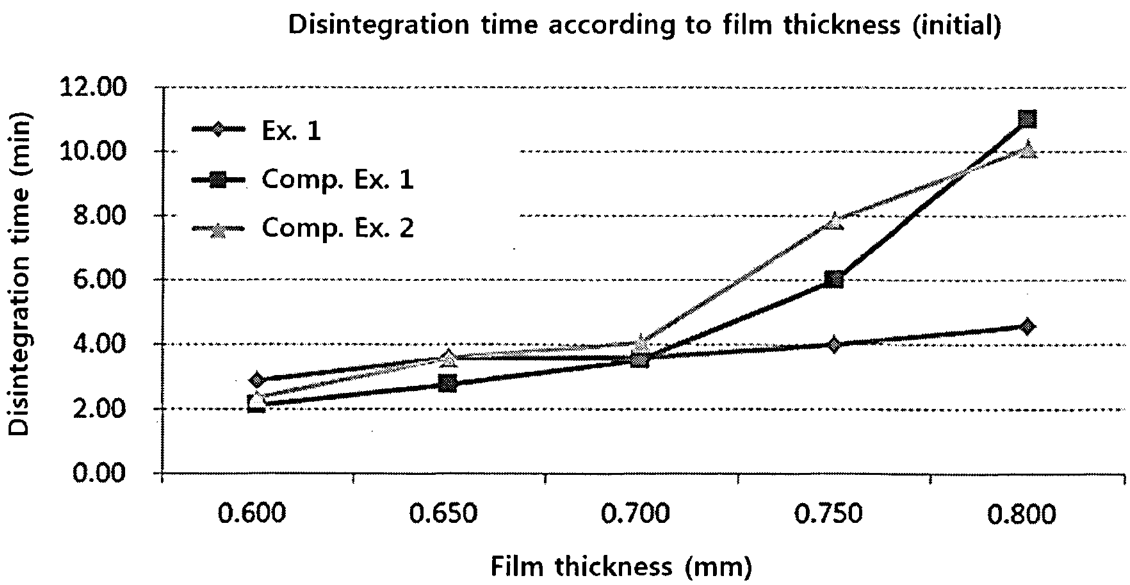
Disintegration time according to film thickness (initial)
Disintegration time (min)
12.00
10.00
8.00
6.00
4.00
2.00
0.00
0.600
0.650
0.700
0.750
0.800
Film thickness (mm)
Ex. 1
Comp. Ex. 1
Comp. Ex. 2

Fig. 3

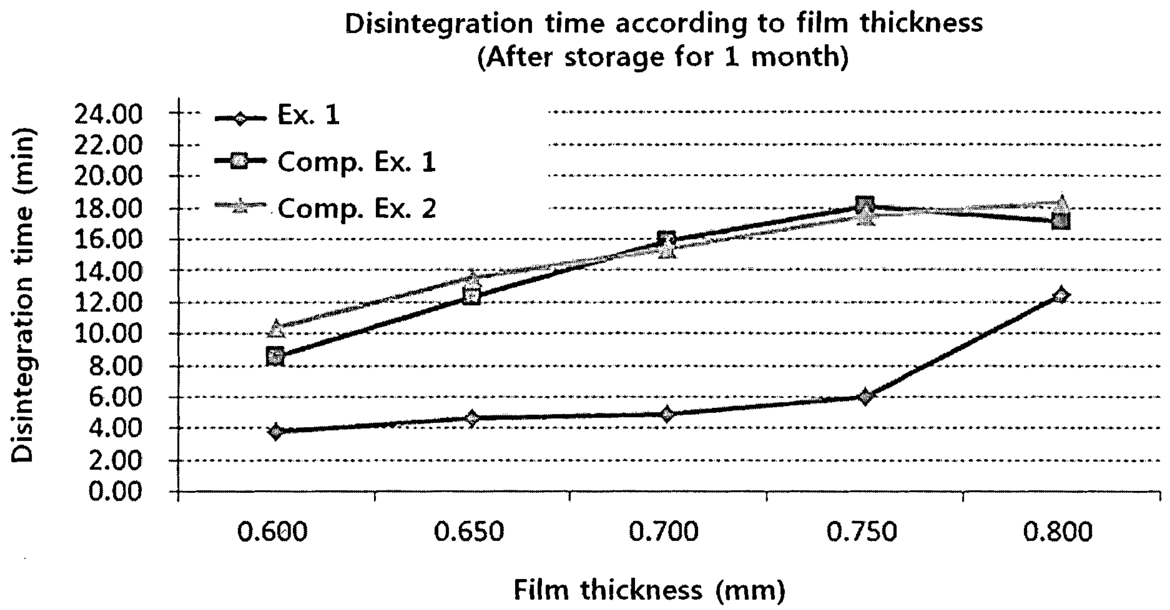
Disintegration time according to film thickness
(After storage for 1 month)
Ex. 1
Comp. Ex. 1
Comp. Ex. 2
Disintegration time (min)
24.00
22.00
20.00
18.00
16.00
14.00
12.00
10.00
8.00
6.00
4.00
2.00
0.00
0.600
0.650
0.700
0.750
0.800
Film thickness (mm)

Fig. 4A

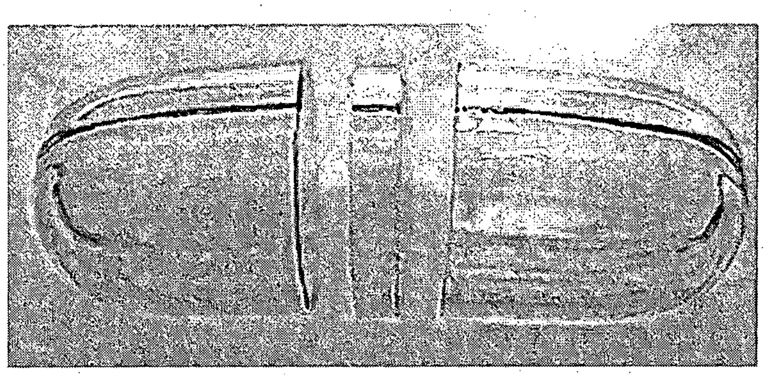

Fig. 4B

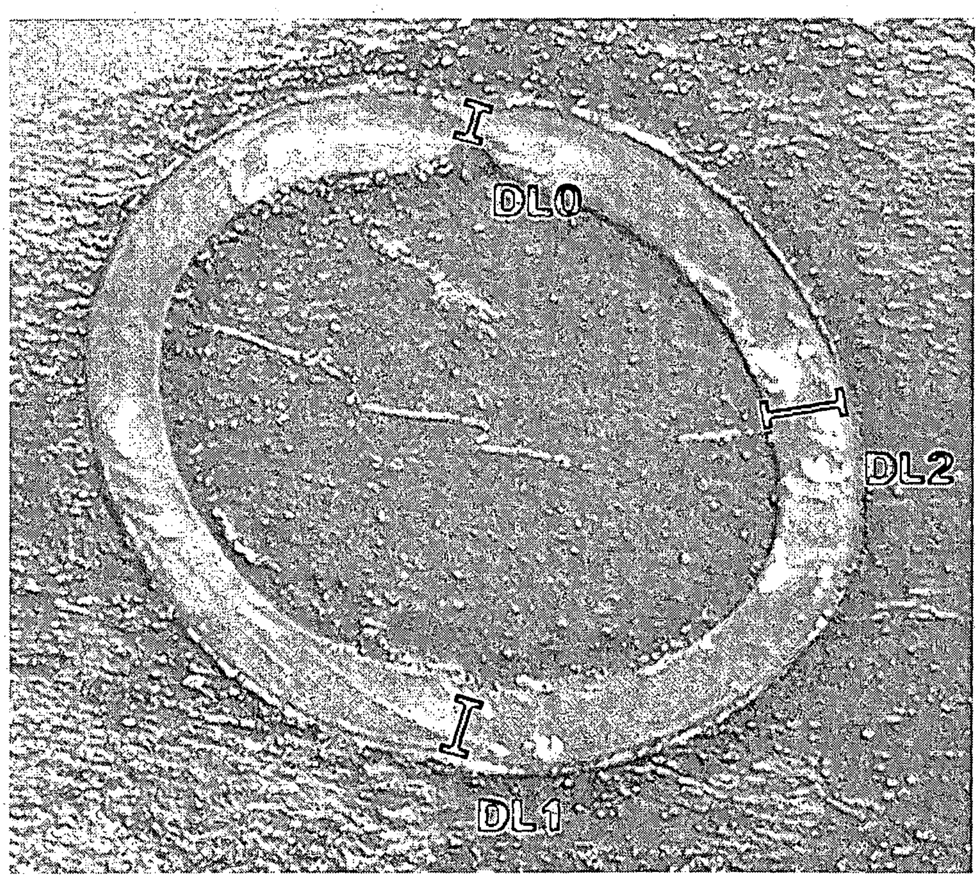

Fig. 5

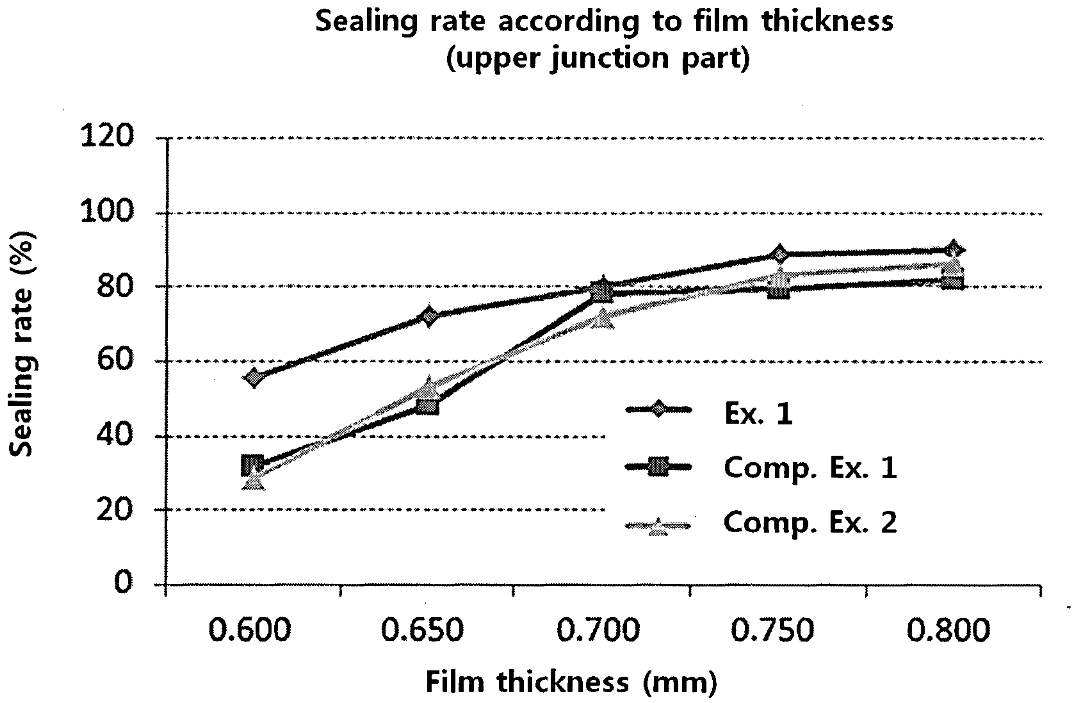
Sealing rate according to film thickness
(upper junction part)
Sealing rate (%)
120
100
80
60
40
20
0
0.600
0.650
0.700
0.750
0.800
Film thickness (mm)
Ex. 1
Comp. Ex. 1
Comp. Ex. 2

Fig. 6

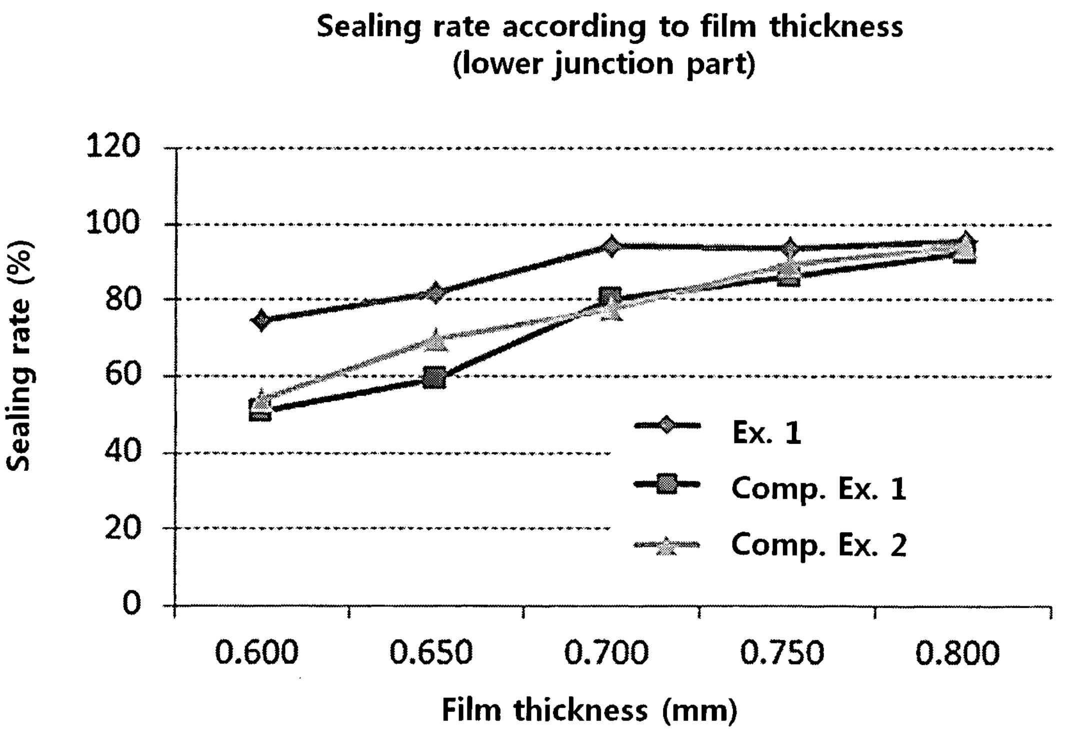
Sealing rate according to film thickness
(lower junction part)
Sealing rate (%)
120
100
80
60
40
20
0
0.600
0.650
0.700
0.750
0.800
Film thickness (mm)
Ex. 1
Comp. Ex. 1
Comp. Ex. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5565467 A
- KR 101055412
- KR 1020050030282
- WO 2010092596 A1
- KR 200901300580 A


### Non-patent literature cited in the description


- AVODART SOFT CAPSULES 0.5 MG 1. NAME OF THE MEDICINAL PRODUCT. Glaxosmithkline, 13 March 2013, 1-16